# EUROPEAN PATENT SPECIFICATION

(11) **EP 3 004 064 B1**
(45) Date of publication and mention of the grant of the patent: **01.11.2017**
(21) Application number: 13742505.4
(22) Date of filing: 06.06.2013
(51) Int. Cl.: C07D 235/28, A61K 31/4184, A61P 31/04, A61P 31/12

(54) **PHARMACEUTICAL COMPOSITIONS BASED ON PHOTOCHEMICALLY STABLE SILVER COMPLEXES, CHLOREXIDINE AND CATIONIC SURFACTANTS**
PHARMAZEUTISCHE ZUSAMMENSETZUNGEN AUF BASIS VON PHOTOCHEMISCH STABILEN SILBERKOMPLEXEN, CHLOREXIDIN UND KATIONISCHEN TENSIDEN
COMPOSITIONS PHARMACEUTIQUES À BASE DE COMPLEXES D'ARGENT PHOTOCHIMIQUEMENT STABLES, DE CHLORHEXIDINE ET DE TENSIOACTIFS CATIONIQUES

(43) Date of publication of application: 13.04.2016
(73) Proprietor: Pavia Farmaceutici S.r.l., 27010 Copiano (Pavia) (IT)
(72) Inventor: FERRARI, Massimo, I-27010 Copiano (PV) (IT)
(74) Representative: Biggi, Cristina
(86) International application number: PCT/IB2013/054649
(87) International publication number: WO 2014/195763

(56) References cited:
- WO-A2-2006/015317
- JP-A- 3 120 532
- DATABASE CA [Online] CHEMICAL ABSTRACTS SERVICE, COLUMBUS, OHIO, US; SUPRUNOVICH, V. I. ET AL: "Study of complexing of silver with 8-mercaptoquinoline. Composition and stability of mixed thiooxinates", XP002717330, retrieved from STN Database accession no. 1979:563850
- BATTISTUZZI, RAFFAELE ET AL: "Silver(I) complexes of 4,6-dimethylpyrimidine-2(1H)-thione", CANADIAN JOURNAL OF CHEMISTRY , 59(3), 591-6 CODEN: CJCHAG; ISSN: 0008-4042, 1981, XP002717331, DOI: 10.1139/V81-086

## Description

The present invention generally relates to photochemically stable silver complexes of formula (I) and (II), and to compositions containing them, useful as antimicrobial, antiviral or antimycotic agent.

### Background art

The use of topical antimicrobial agents for wound care gained a wide acceptance in the 1960s once it was discovered that treating burns with silver nitrate decreased the number of deaths that were a result of sepsis from 60% to 28%.

Antiseptic silver sulfadiazine (SSD) was associated with additional decreases in infection, eventually making a place for itself in general wound care. Silver sulfadiazine demonstrated improved outcomes and decreased infection rates.

Antiseptics differ from antibiotics in that they have broad-spectrum activity and can be effective against many types of organisms including aerobic and anaerobic bacteria, yeasts, fungi, and molds.

Bacterial resistance to antibiotics is extensively documented in medical literature. Resistance to antiseptics, however, has only been studied more recently. Some authors compared the ability of Staphylococcus epidermidis to develop resistance to various antibiotics and antiseptics. Results suggested that the bacteria developed resistance to the antibiotics minocycline and rifampicin, however no evidence of resistance was observed with chlorhexidine, silver sulfadiazine and polyhexametylene biguanide.

Silver has been used as an antimicrobial agent for thousands of years. The activity of silver lies mainly in its ionic form, thereby silver ions (Ag⁺) exert varying antimicrobial effects, typically depending on their binding site. For instance, when binding occurs at the bacterial cell wall, ruptures can occur. When bound to proteins involved in respiration and nutrition of the organism, silver can block these processes with the consequent elimination of the bacteria. When binding DNA, silver can affect the replication and division of the organism. Silver ion is an effective antimicrobial agent with low toxicity, which is important especially in the treatment of burn wounds where transient bacteremia is prevalent and its fast control is essential. It has been proved that the monovalent Ag⁺ ion shows a rather broad spectrum of bactericidal activity.

Elemental silver and silver salts demonstrate substantially less effectiveness against microbes. Previously, silver salt solutions, such as silver nitrate, were used to bathe the wound. However, this application requires large amounts of substance to achieve the desired effect. Silver sulfadiazine (SSD) creams enable the use of much lower amounts to be effective, and they act by discharging silver ions when in contact with wound exudate. The silver ions, however, may be rapidly neutralized, thereby requiring daily, or even more frequent, application of SSD. The amount of silver released into the wound is not always clearly defined and can be a concern for toxicity in healthy tissue. Ag⁺ based formulations are thus applied in pharmaceutical preparations, specially for topical use, as well as in disinfectants formulations, e.g. for applications on daily objects surfaces and the like. However, most of the formulations based on silver salts, e.g. silver sulphadiazine, suffer of photochemical instability and, particularly, of low water solubility. Also for this reasons, colloidal silver nanoparticles have been the focus of increasing interest, and are being heralded as an excellent candidate for therapeutic purposes. The term "colloidal" refers to a substance comprising a medium having ultra-fine particles that do not dissolve in it, thus remain unsolved, typically as suspension, depending e.g. on the nature and density of the selected medium. The silver ion particles are electronically charged to activate the germicidal activity of the silver also allowing the particles to remain suspended in a deionized water medium. Such colloidal preparations are, however, photochemically unstable, to such an extent that the exposure to visible light can cause the photoreduction of Ag⁺ to Ag° with a consequent breakdown of the antimicrobial action. The production of Ag⁺ ions from metallic Silver (Ag°), is in addition limited by the high oxidation potentials of Ag° to Ag⁺ (E1/2 = + 0.80 V vs normal Hydrogen Electrode, at 25°C) which implies an equilibrium constant for silver oxidation in the order of 1.2 x 10⁻⁶, indicating that 1 M of Ag° (107.8 g) can produce, at the equilibrium, only 1 x 10⁻⁴ g of Ag⁺.

Considering that Ag⁺ is active against over 600 different types of Bacteria, Fungi and Viruses the search for new and superior in stability silver-based antimicrobial agents still remain a technical problem to be solved. WO 2006/015317 discloses antimicrobial agents.

### Summary of the invention

In a first aspect, the invention relates to an anionic monovalent silver complex of general formula (I), and to a cationic silver complex of general formula (II):

**Ag⁺---L₁⁻M⁺]** **(I)**

**Ag⁺---[L₂H⁺]⁺Y⁻** **(II)**

wherein:
in formula I:
   **[_{L1}⁻ M⁺]** is a moiety of general formula: 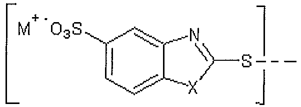
wherein:
- **X**: is selected from the group consisting of -NH-, -O-,-S- and -NR-, wherein R is a C₁-C₆ linear or branched alkyl group (C₁-C₆Alk) , or a carbonyl-C₁-C₆ linear or branched alkyl group (-C(O)-C₁-C₆Alk), preferably acyl group (-C(O)-CH₃), and
- **M⁺**: is hydrogen ion (H⁺) or an alkali metal ion, preferably sodium ion (Na⁺),
and wherein in formula II:
- **Y⁻**: represent a counter anion, preferably selected from: acetate, nitrate, lactate, trifluoromethane, chloride, bromide and sulphonate;
- **[L₂H⁺]⁺**: is the protonated form of the ligand moiety, selected from: 2-mercapto-4-(C₁-C₆Alk)-pyrimidine, preferably 2-mercapto-4-methylpyrimidine, 2-mercapto-4-(C1 -C6 Alk) pyrimidine hydrochloride, 8-mercapto-quinoline hydrochloride, preferably 2- mercapto-4-methylpyrimidine hydrochloride.
wherein in both formulae (I) and (II) the Ag⁺ ion is coordinated to the thiolic sulfur atom of [L₁⁻M⁺] and [L₂H⁺] respectively, as schematically indicated by the dotted lines.

In a preferred embodiment, the complex (I) and (II) are prepared in an aqueous medium, thus being in the form of an **aqueous solution.**

In one embodiment, said aqueous solution also contains at least one additional antimicrobic, antiviral, and/or antimycotic agent, thus forming an **aqueous formulation.**
In this direction, preferred agents are: chlorexidine, either acetate or gluconate salt, and/or didecyldimethylammonium cations, being the mixture of chlorexidine, acetate or, preferably, gluconate salt, and didecyldimethylammonium chloride particularly preferred.

In an additional aspect, the invention also refers to a **kit of parts** for the preparation of the complex (I) or (II) in form of the above indicated aqueous solution or formulation.

Another object of the invention is a **pharmaceutical composition** comprising one complex of the above reported formula (I) or (II), in admixture with at least one physiologically acceptable carrier or excipient, and/or eventually also in admixture with at least one antimicrobial, antiviral or antimycotic agent.

A further object of the invention is a **process for the preparation** of a complex of formula (I) and (II), said process comprising the steps of:
a) dissolving in water the [L₁⁻M⁺] or the [L₂H⁺]Y⁻ moiety;
b) adding a proper amount of Ag⁺ ion, preferably in the form of silver nitrate.

In an additional aspect, the invention relies on a complex of formula (I) and (II), e.g. in the form of aqueous solution or formulation, **for use as a medicament,** particularly as antimicrobial, antiviral or antimycotic agent.
In this respect, in a preferred embodiment, the invention refers to pharmaceutical composition for the topical administration, comprising the present derivative in admixture with at least one physiologically acceptable excipient or carriers, even more preferably in the form of a cream or a gel.
A still further object is the **non-medical use** of the complexes of formula (I) and (II), as disinfectants, in particular for the treatment of surfaces of objects and paper materials.

### Detailed description of the invention

The term "linear or branched C₁-C₆ alkyl group" or "C₁-C₆-Alk", means a linear or branched hydrocarbon chain residue, having from 1 to 6 carbon atoms, e.g.: methyl, propyl, isopropyl, butyl, isobutyl, ter-butyl and the like.
Similarly, the term "carbonyl-C₁-C₆ linear or branched alkyl group" means a C₁-C₆ alkyl group connected to the nitrogen atom of the X group through a carbonyl group (-C(O)-).
Unless otherwise provided, the term "alkali metal ion" means a positive ion derived from a metal of the Group I of the periodic table, such as: sodium, potassium lithium and the like.
As herein intended, the term "aqueous solution" means an aqueous medium wherein the complex (I) or (II) have been prepared, typically without remaining suspended, differently from what happens for instance in the colloidal preparations. In one embodiment of the invention, the aqueous solutions have a concentration from 10⁻⁶ M to 10⁻¹ M, being value comprised from 10⁻⁴ M and 10⁻² M particularly preferred.
The term "aqueous formulation" indicates an aqueous solution comprising either a complex (I) or (II) in admixture with at least one additional antiviral, antimicrobial, antimycotic agent.

An aspect of the present invention is a monovalent anionic or cationic silver complex, respectively of general formula (I) and (II):

**Ag⁺---[L₁⁻ M⁺]** **(I)**

**Ag⁺---[L₂H⁺]⁺ Y⁻** **(II)**

wherein [L₁⁻M⁺], [L₂H⁺], and Y⁻ are as above defined.
The present complexes are characterized by having a high stability in aqueous solutions, whereby they are endowed with a prolonged photochemical and thermal stability. This allows the preparation and the storage of aqueous solutions or aqueous formulations comprising them, useful as antimicrobial, antiviral and antimycotic agent, as herein below described in detail, and further supported by the enclosed experimental part.
According to an embodiment of the invention, the present photochemically stable monovalent silver complexes have the following general formula (I):

**Ag⁺---[L₁⁻ M⁺]** **(I)**

wherein:
M⁺ is a hydrogen ion (H⁺), or an alkali metal ion, preferably Na⁺.
The ligand L₁ is a moiety which is able to coordinate the silver atom ion (Ag⁺), thus forming the complex Ag⁺---[L₁⁻M⁺]. In this respect, L₁ is preferably selected from the group consisting of: 2-mercapto-5-benzoimidazole sulfonic acid (L'), 2-mercapto-5-benzoimidazole sulfonic acid sodium salt (L") and 2-mercapto-5-benzooxazole sulfonic acid (L'''), having the following chemical formulae: 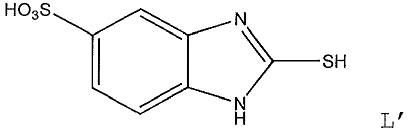 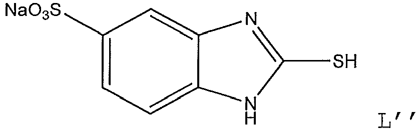 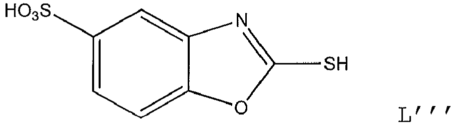 The present complex of formula (I) can also be represented by the following general formula: 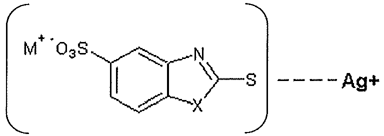 wherein the dotted line indicates the coordination bond between the silver ion and the mercapto group (sulfur atom) of the [L₁⁻M⁺] moiety, and wherein X and M⁺ are as above defined.

The complexes of formula (I) can be prepared e.g. by dissolving in water the moiety [L₁⁻M⁺], i.e. the thiolic sulfonic ligand in a protonated or as alkaline salt thereof, of formula: 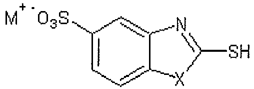 wherein M⁺ and X are as above defined.
By that, it is possible to obtain a complete dissociation of the sulfonic acid group (-SO₃H), or of the corresponding sodium salt, to form the anionic L₁⁻M⁺ species containing the anionic (-SO₃⁻) functionality.
Addition to this solution of Ag⁺ ions, preferably in a stoichiometric amount, results in the formation of the water soluble metal complex of formula (I), wherein the Ag⁺ ion is linked to the sulfur atom of the mercapto or thiolic group (-SH), thus replacing the proton.
Preferably, the silver ions are added in form of a water soluble salt, even more preferably silver nitrate (AgNO₃). The thus obtained complex (I) could be used in water solution or alternatively can be collected in solid form, e.g. by precipitation and/or crystallization using procedure known in the art, such as addition of a crystallization solvent like the ethanol/diethylether system.
The above procedure can be applied also for the preparation of complexes of formula (II).
Therefore, both the complexes of the formulae (I) and (II) can be obtained in aqueous solution or even isolated as powder, or formulated as tablets, or similar solid forms, or in any form suitable for the administration in any conventional way to a patient in need thereof, being the topical administration, e.g. *via* cream or gel formulation, particularly preferred as herein above explained in detail. In an equally preferred embodiment, the photochemically stable cationic silver complexes according to the invention have the following general formula (II):

**Ag⁺---[L₂H⁺]⁺ Y⁻** **(II)**

wherein:
- Y⁻: represent a counter anion, preferably selected from: acetate, nitrate, lactate, trifluoromethane, chloride, bromide and sulphonate;
- [L₂H⁺]⁺: is the protonated form of the ligand moiety, selected from: 2-mercapto-4-(C₁-C₆-Alk)-pyrimidine, preferably 2-mercapto-4-methylpyridine.
Preferably, in the present formula (II), the moiety [L₂H+]⁺Y⁻ is selected from 8-mercapto-quinoline hydrochloride, and 2-mercapto-4-(C₁-C₆-Alk)-pyrimidine, preferably 2-mercapto-4-methylpyrimidine, of formula: 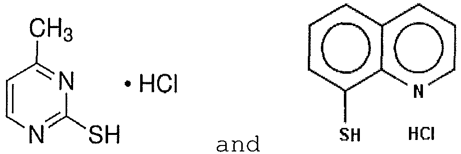 In a more preferred embodiment, in the complex of formula (II), the counter ion Y⁻ is Chloride.
The present complex (I) and (II) are also characterized by the fact that the Ag⁺ ion is coordinated to the thiolic (or mercapto) sulfur group of the ligand L₁ or L₂ as above explained. In this direction, stability tests confirmed that both thermal and photochemical stability of the complexes of formula (I) and (II) was surprisingly obtained, even when they are dissolved in water, thus being obtained in the form of aqueous solutions or formulations thereof. Advantageously, the present invention provides a complex of formula (I) or (II) which can enhance the stability over the time of the Ag⁺ ion, thus overcoming one of the key problem generally associated to the use of the silver ions in the medical field, namely the low stability of the Ag⁺ ions. In fact, due to their high stability, the aqueous solution or formulation of the invention can be prepared beforehand, e.g. well before their application, and safely stored even for a long frame of time. The Applicant has noted that aqueous solutions or formulations comprising the complexes of the invention (either (I) or (II)) did not show any substantial change, e.g. in composition or in color, even after exposure to solar (or artificial) light for more than 12 months. In comparable conditions, commonly known aqueous solutions of silver salts, such as nitrate, acetate, perchlorate, hexafluorophosphate, or the like, show, on the contrary, formation of a black Ag° precipitate just after few minutes, symptomatic of the degradation of the silver salt used.
The present aqueous solutions can be obtained by preparing the complex of formula (I) or (II), in a proper aqueous medium, preferably selected from: sterile water, physiological saline (i.e. sterile water solutions of NaCl), ultra pure water, deionized water, distilled water and water for injection (WFI).
In a still preferred embodiment, said aqueous solution also comprises at least one antimicrobic, antiviral or antimycotic agent, thus forming an aqueous formulation thereof. In this respect, the present invention also provides an aqueous formulation obtained by dissolving a complex of formula (I) or (II) in a proper aqueous medium, e.g. sterile water, containing at least one additional antimicrobial, antiviral and/or antimycotic agent. Therefore, according to an additional embodiment, the invention refers to an aqueous formulation comprising: a complex of formula (I) or (II) in admixture with at least one, preferably two, antiviral, antibacteric and/or antimycotic agent.
In a particular preferred embodiment, said aqueous formulation comprises the present complex (I) in admixture with Chlorexidine or gluconate or acetate salt thereof and/or didecyldimethylammonium cations. Preferred didecyldimethylammonium cations are: chloride, bromide and the like. Even more preferably, the formulation comprises the present complex (I) in admixture with Chlorexidine gluconate and didecyldimethylammonium chloride, of general formula:

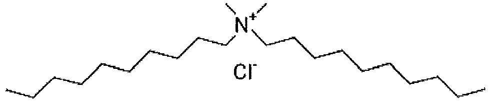

Equally preferred are aqueous formulations comprising the complex of formula (II) in admixture with chlorexidine, more preferably as gluconate or acetate salt of formula: 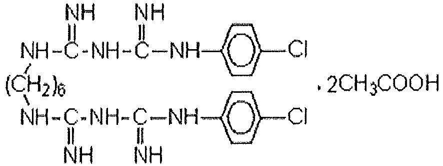 and

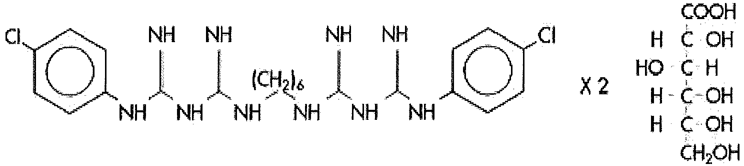

The applicant has found that when at least one additional antiviral, antimicrobic or antimycotic agent is added to the present aqueous solution, it is possible to obtain a synergic and improved effect of the components, as clearly demonstrated by the following experimental part. In the presence of the silver complex (I) or (II), the resulting formulation, in fact, shows an enhanced microbial activity, with respect to the same formulation but devoid of the silver complex (I) or (II).
Also, the high stability of the thus obtained aqueous solutions and/or formulation is particularly useful in that it is now possible to prepare and safely store antimicrobial silver ions derivatives, with improved pharmaceutical activity. Even further, due to the high stability behavior, complexes (I) and (II) are useful even in the manufacture of antiviral, antibacterial or antimycotic medicaments, e.g., in form of medical devices. As far as the amount of the components is concerned, the silver complex can be used in a ratio %W/v from 0.003 to 0.0001, preferably, from 0.03 to 0.001, the chlorexidine can be used in a ratio from 0.1 to 1 w/v%, preferably from 0.2 to 0.6%, whereas the didecylammonium cation can be used from 0.2 to 2 w/v%, preferably from 0.5 to 1.5 w/v%.
The term "w/v%" indicates the weight of the substance over the total volume of the solution.
The present formulations can be prepared e.g. by solving the complex (I) or (II) in a selected aqueous medium, wherein the additional agent has been previously solved. Alternatively, the formulation can be obtained by premixing the complex (I) or (II) with the selected agent, followed by the dissolution of the thus obtained mixture in the proper aqueous medium. In both cases the aqueous medium can be selected as above indicated.

In one further aspect the invention refers to a complex of general formula (I) or (II) for use as a medicament, particularly as antimicrobic, antiviral or antimycotic agents, more preferably in the form of an aqueous solution, aqueous formulation or pharmaceutical compositions for topical administration.
Preferably, the present complex (I) or (II), e.g. as aqueous solution, formulation or topical composition, is for the use as antibacteric agent, more preferably against the following bacteria: *Legionella pneumophila, Pseudomonas aeruginosa, Staphilococcus aureus, Enterococcus faecalis, Escherichia coli, Salmonella enteridis D1, Listeria monocytogenes, Neisseria gonorrhoeae, Neisseria meningitidis*,*Vibrio cholerae, MRSA, Clostridium Diff, Acinetobatcter Baumanii, Mycobacterium terrae, Mycobacterium avium and Bacillus subtilis.*
In a further embodiment, the present complex (I) or (II), e.g. as aqueous solution, formulation or topical composition, is for the use as antimycotic agent, preferably against: *Candida albicans and Aspergillus niger fungi.*
In a further embodiment, the present complex (I) or (II), e.g. as aqueous solution, formulation or topical composition, is for the use as antiviral agent, preferably against the following Viruses: *A-H1N1, Adenovirus, Poliovirus, Citomegalovirus, Enterovirus, Herpes virus, Hepatitis A Virus, Hepatitis B Virus, Human Immunodeficiency virus (HIV), Varicella Zoster Virus, Aviaria Viruses Group and SARS Corona Virus.*
Complexes (I) or (II) are intended for the topical use as such or, for instance, adsorbed on a proper medical support, e.g., bandages, patches and the like. Also, and according to an embodiment of the invention, the present complexes (I) or (II) are formulated in the form of topical composition, in admixture with physiologically acceptable carriers or excipient, being thus useful also as medical devices.
Therefore, in one further aspect, the invention relates to a pharmaceutical composition comprising the monovalent silver complexes (I) or (II), in admixture with at least one physiologically acceptable carrier or excipient, and/or preferably in admixture also with at least one antimicrobial, antiviral or antimycotic agent, even more preferably selected from: chlorexidine digluconate or acetate, didecyldimethylammonium cations and, still more preferably, mixtures of chlorexidine digluconate and didecyldimethylammonium chloride. In particular, a pharmaceutical composition for topical use will be preferred. This composition will be preferably in form of a liquid or a semi-liquid product selected from: solutions, suspensions, lotions, ointments and, preferably, a cream or a gels.
The pharmaceutical compositions of the invention can be prepared according to conventional methods, such as the ones described in Remington's Pharmaceutical Sciences Handbook, Mack Pub. Co., N.Y., USA, 17th edition, 1985. Conveniently, the physiologically acceptable carriers and/or excipients of the present composition, can be selected among those commonly used in the art. As general examples, when the composition is in the form of a cream, said excipients can be suitably selected e.g. from: vaseline, liquid paraffin, stearyl alcohol, polyethylene glycol stearate and the like.
Similarly, if the composition is in the form of a gel, the present derivatives of formula (I) can be admixed with, e.g., glycerine, amidopropylene glycol, and the like.

In an additional aspect, the invention refers to the non-medical use of the present complex (I) or (II), preferably in the form of aqueous solution or formulation as above described in details, as disinfectant agent for the treatment of the surfaces of objects of various nature, particularly domestic or medical objects. As an example, the formulation of the invention can be used to disinfect floors, walls, furniture, apparatuses, devices, particularly in a medical environment such as an hospital or the like.

In an additional aspect, the invention also refers to a kit of parts for the preparation of the complex (I) or (II) in form of an aqueous solution, separately comprising at least:
- the present complex of formula (I) and/or (II), and
- a container, such as a vial, containing a proper amount of the aqueous medium, preferably water for injection or saline.
In a further embodiment, the kit also contains at least one additional antimicrobic, antiviral or antimycotic agent, preferably selected as above indicated, either separate or even mixed with the selected complex of formula (I) or (II). Alternatively, said at least one additional agent can be dissolved in the selected aqueous medium. In these latter cases, the kit is thus useful for the preparation of the above indicated aqueous formulations.

As clearly described above in details, the present invention provides new complexes of formula (I) and (II) useful in particular as antimicrobic agents, due to the presence of Ag⁺ ions. Of note, even when formulated as aqueous solutions, alone or in combination with additional active ingredient as afore mentioned, they are characterized by an improved shelf life and long photostability. Even further, when the complexes (I) or (II) are admixed in pharmaceutical compositions or just in aqueous solutions with at least one antimicrobial, antiviral or antimycotic agent, they can exert a multi tasking composition, being able to synergically combine the activity of Ag⁺ ions with the one owned by the chosen agent.
The invention will be now described by way of examples, without limiting its scope.

### EXPERIMENTAL PART

### EXAMPLE 1: preparation of the anionic complex Ag-L₁⁻, wherein L = 2-Mercapto-5-benzimidazolesulfonic acid sodium salt (Ag⁺--- [L⁻Na⁺]).

The anionic complex Ag-L₁⁻ with L = 2-Mercapto-5-benzimidazolesulfonic acid sodium salt was prepared by addition of the ligands L to an aqueous solution containing a stoichiometric amount of AgNO₃.
The product, formed instantly, was precipitated by addition of ethanol/diethyl ether and the solid dried at 50 °C for 12 h in a ventilated oven.

### Chemical characterization

### Elemental analysis of Ag⁺---[L⁻Na⁺] of Example 1

Calculated for NaAgS₂O₃H₄C₇N₂ (Mol. Weight = 359.11) : C, 23.41; N, 7.80; H, 1.12; S, 17.86.
Found: C, 23.39; N, 7.82; H, 1.09; S, 17.82.
In addition to elemental analysis data, which are fully consistent with the formula of the complex, Infrared spectroscopy (IR) data confirm the coordination of the Silver ion to the Sulfur atom of the organic ligand. The uncoordinated free ligand, in fact, exhibits IR bands at 2576 cm⁻¹, which can be confidently assigned to the corresponding S-H stretching modes. Upon coordination of Silver ions and binding of Silver to the Sulfur end of the ligand, such band disappear leaving unmodified the other parts of the IR spectrum. The disappearance of the S-H stretching band confirms that binding of Silver ions to Sulfur has occurred. According to Sandroff et al. J. Phys Chem. 1992, 86, 3277 *,* the S-Ag stretching band should be expected in the frequency range 150-250 cm⁻¹ which is however beyond the instrumental limit of the used IR spectrometer (4000-400 cm⁻¹).
Stability tests did not show any change in color or composition after 12 months to ambient light exposure. In comparable conditions, solutions of Silver salts, such as nitrate, acetate, perchlorate or hehafluorophosphate, showed formation of a black Ag° precipitate after few minutes.

### Example 2: Bactericidal Activity

### Example 2a: Bactericidal Activity of a complex of formula (I) in form of aqueous solution.

The bactericidal activity was tested on aqueous solutions of the complex of Example 1, (i.e. where the ligand L coordinated to the silver ion is the 2-Mercapto-5-benzimidazolesulfonic acid sodium salt).

The bactericidal activity tests were carried out according to UNI EN 1272:2009 and UNI EN 13697:2001 standards, which makes reference to chemical disinfectants and antiseptics, by using the following test organism: *Staphylococcus aureus* (ATCC- 6538), as representative of Gram-positive bacteria and *Pseudomonas aeruginosa* (ATCC - 15442), as representative of Gram-negative bacteria.
According to UNI EN 1272:2009, which is a quantitative suspension test for the evaluation of bactericidal activity, it was found that the present silver complex of (where L = 2-Mercapto-5-benzimidazolesulfonic acid sodium salt) in concentration of the order of 6.6 x 10⁻⁴ M, in water, was able to cause a 1.2 log reduction of bacterial count after 5 minutes of contact and a 3 log reduction of bacterial count after 30 min of contact, analogously to what observed for a Silver Nitrate solution of comparable concentration, which however was observed to lose the antimicrobial activity after exposure to ambient light, due to reduction of Ag⁺ to Ag°.

### Example 2b: Activity of a complex of formula (I) in form of aqueous formulation.

Evaluation of the bactericidal and fungicidal activity of aqueous formulations containing the complex of Example 1 [Ag-L⁻]Na, where L⁻Na = 2-Mercapto-5-benzimidazolesulfonic acid sodium salt), Didecyldimethylammonium Chloride and Chlorexidine Digluconate was performed according to UNI EN 1276, April 2000,standard suspension test.
The suspension test was performed on aqueous formulation containing:
- 0.0016% (4.5 x 10⁻⁵ M)of the Silver complex,
- Didecyldimethylammonium Chloride at 1.25% and
- Chlorexidine Digluconate at 0.25%, indicated as product solution (A).
Comparative test were also performed on an analogous solution, lacking of the Silver complex and containing Didecyldimethylammonium Chloride at 1.25% and Chlorexidine Digluconate at 0.25%, indicated as product solution (B). The antimicrobic activities of the solutions were tested against the following microbic species,

| | |
|---|---|
| *Pseudomonas aeruginosa* | ATCC 15442 |
| *Staphylococcus aureus* | ATCC 6538 |
| *Escherichia coli* | ATCC 10536 |
| *Enterococcus hirae* | ATCC 10541 |
| *Candida albicans* | ATCC 10231 |

which were purchased from Diagnostic International Distribution SpA. Test microorganism suspensions were adjusted in the concentration range 1.5x10⁸ - 5.0x10⁸ cfu/ml.

Test solutions of the products, test microrganisms suspensions, interfering substance, tryptonated and distilled water were previously stabilized at the test temperature of 20°C. For each bacterial stock and for each product, a test tube containing 1 ml of interfering substance and 1 ml of test organism suspensions in the range 1.5x10⁸ - 5.0x10⁸ cfu/ml were mixed. After 2 minutes, 8 ml of the solution containing the product was mixed and left in contact for 5 minutes at 20°C. 1 ml of this solution was then transferred in a test tube containing 8 ml of tryptonated water and 1 ml of distilled water and shacked. Finally 1 ml of this mixture was transferred in a Petri capsula and after an incubation time of 48 hours, at 37°C ± 1°C, the number of cfu / plate was determined.
The vitality reduction values (R) for the different microorganism which have been in contact with the two product solutions for 5 minutes are reported in Table I. The results shown in table I demonstrate that in 5 min of contacts with the antimicrobic aqueous formulation containing the silver complex, Didecyldimethylammonium chloride and Chlorexidine a vitality reduction higher that 5 log is observed while in the absence of the silver complex the activity was limited to ca 4 log. Applicants would like to note that at the concentration level of 4.5 x 10⁻⁵ M and in 5 minutes of contact the silver complex is almost inactive towards the bacterial pull, as a consequence, the superior activity observed for the formulation containing the silver complex, Didecyldimethylammonium chloride and Chlorexidine suggest a synergic action between the components.

**Table I: comparative results.**

| | **Vitality Reduction (R) in the presence of the different product solutions** | |
|---|---|---|
| ***Micro-Organism*** | **(R)** | |
| | **A** | **B** |
| ***Staphylococcus* aureus** | 9.7x10⁵ | 8.1x10⁴ |
| ***Pseudomonas aeruginosa*** | 7.7x10⁴ | 6.2x10³ |
| ***Escherichia coli*** | 1.2x10⁵ | 0.8x10⁴ |
| ***Enterococcus hirae*** | 2.3x10⁵ | 2.0x10⁴ |
| ***Candida albicans*** | 7.8x10⁴ | 6.0x10³ |

This is further supported by surface test performed according to UNI EN 13697:2001, carried out in presence of bovine serum albumin at 0.3%, which is known to deactivate the antimicrobial action of quaternary ammonium salts. The results of these tests showed that after 5 minutes of contact, with the above indicated bacterial pool, the silver complex at concentration of 6.6 x 10⁻⁴ M was able to cause a reduction of the bacterial count of ca 1 log, while a reduction of ca 2 log was observed for the solution containing Didecyldimethylammonium Chloride at 1.25% and Chlorexidine Digluconate at 0.25%. A much higher activity, with a ca 5 log reduction of the bacterial count was instead observed for the solution containing all species and where, for purpose of comparison, their original concentrations where maintained.

### Example 3: bactericidal and fungicidal activity of aqueous solutions containing: [Ag-L₂H+]⁺Cl⁻ (L₂H+]⁺Cl⁻ = 2-mercapto-4-methylpyrimidine hydrochloride), and chlorexidine

Test related to the titled aqueous formulation also indicated a superior antimicrobic activity with respect to aqueous solutions containing only chlorexidine at the same concentration level, consistent with the previously discussed synergy of the components.

## Claims

1. A silver complex of general formula (I) or (II):
Ag⁺---[L₁⁻ M⁺] (I)
Ag⁺---[L₂H⁺]⁺Y⁻] (II)
wherein:
in formula I:
[L₁⁻ M⁺] is a moiety of general formula: 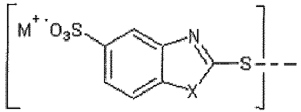 wherein:
X is selected from the group consisting of -NH-, -O-, -S- and -NR-, wherein R is a C₁-C₆ linear or branched alkyl group C₁-C₆Alk, or a carbonyl-C₁-C₆ linear or branched alkyl group -C(O)-C₁-C₆Alk, preferably acyl group -C(O)-CH₃,and
M⁺ is hydrogen ion or an alkali. metal ion, preferably sodium ion Na⁺
and wherein in formula II:
Y⁻ represents a counter anion, preferably selected from: acetate, nitrate, lactate, trifluoromethane, chlorate, bromide and sulphonate;
[L₂H⁺]⁺ is the protonated form of the ligand moiety, selected from: 2-mercapto-4-(C₁-C₆-Alk)-pyrimidine, preferably 2-mercapto-4-methylpyrimidine, 2-mercapto-4-(C₁-C₆Alk) pyrimidine hydrochloride, 8-mercapto-quinoline hydrochloride, preferably 2-mercapto-4-methylpyrimidine hydrochloride.
wherein in both formulae (I) and. (II) the Ag⁺ ion is coordinated to the thiolic sulfur atom of [L₁⁻ M⁺] and [L₂H⁺]⁺ respectively.

2. Complex of formula (I) according to claim 1, wherein L1 is selected from the group consisting of:
2-mercapto-5-benzoimidazole sulfonic acid, 2-mercapto-5-benzoimidazole sulfonic acid sodium salt and 2-mercapto-5-benzooxazole sulfonic acid.

3. Complex of formula (I) or (II) as defined in any one of the preceding claims, in the form of an aqueous solution.

4. Complex of formula (I) or (II) in the form of aqueous solution according to claim 3, obtained by dissolving the complex (I) or (II) of claims 1-3 in an aqueous medium selected from: sterile water, physiological saline, ultra pure water, deionized water, distilled water and water for injection (WFI).

5. Complex of formula (I) or (II) in the form of aqueous solution according to claim 3 or 4, comprising from 0.003 to 0.0001, preferably, from 0.03 to 0.001 of a complex of formula (I) or (II).

6. Aqueous formulation comprising the complex of formula (I) or (II) in the form of aqueous solution as defined in claims 3-5, and at least one additional antibacteric, antiviral or antimycotic agent, preferably selected from the group consisting of:chlorexidine digluconate or acetate, didecyldimethylammonium cations, preferably chloride, and/or mixtures thereof.

7. Aqueous formulation according to any one of the claims 5 or 6, comprising the complex of formula (I) in admixture with chlorexidine digluconate or acetate, and didecyldimethylammonium chloride.

8. Aqueous formulation according to any one of the claims 5 or 6, comprising the complex of formula (II) in admixture with chlorexidine digluconate oracetate.

9. Aqueous formulation according to any one of the claim 8 comprising: from 0.003 to 0.0001, preferably, from 0.03 to 0.001 w/v% of a complex (I) or (II), chlorexidine in a ratio from 0.1 to 1 w/v%, preferably from 0.2 to 0.6%, and didecylammonium cation from 0.2 to 2 w/v%, preferably from 0.5 to 1.5 w/v%.

10. Pharmaceutical composition comprising the complex of formula (I) or (II) as defined in any one of the claims 1-5, in admixture with at least one physiologically acceptable carrier and/or excipient, and optionally also in admixture with at least one additional antimicrobic, antiviral, antimycotic agent.

11. Pharmaceutical composition according to claim 10, for topical administration.

12. Pharmaceutical composition according to claim 11, in the form of cream, gel or foam.

13. Use of the complex (I) or (II), as defined in any one of the claims 1-5 as disinfecting agent for surfaces, domestic or medical objects.

14. Complex of formula (I) or (II) as defined in any one of the claims 1-5, for use as medicament.

15. Complex of formula (I) or (II) for use, according to claims 13 or 14, as antimicrobic, antiviral or antimycotic agent.

16. Complex of formula (I) or (II) for use, according to claims 14-15 as antibacteric agent against the bacteria selected from: Legionellapneumophila, Pseudomonas aeruginosa, Staphilococcus aureus, Enterococcus faecalis, Escherichia coli, Salmonella enteridis D1, Listeria monocytogenes, Neisseria gonorrhoeae, Neisseria meningitidis,Vibrio cholerae, MRSA, Clostridium Diff, Acinetobatcter Baumanii, Mycobacteriuni terrae, Mycobacteriuni avium and Bacillus subtilis.

17. Complex of formula (I) or (II) for use, according to claims 14-15 as antimycotic agent against: Candida albicans and Aspergillus niger fungi or as antiviral agent against the Viruses selected from: A-H1N1, Adenovirus, Poliovirus, Citomegalovirus, Enterovirus, Herpes virus, Hepatitis A Virus, Hepatitis B Virus, Human Immunodeficiency virus (HIV), Varicella Zoster Virus,Aviaria Viruses Group and SARS Corona Virus.

## Patentansprüche

1. Silberkomplex der allgemeinen Formel (I) oder (II):
Ag⁺---[L₁⁻ M⁺] (I)
Ag⁺---[L₂H⁺]⁺Y⁻] (II)
wobei:
in Formel I
[L₁⁻ M⁺] ein Teil der allgemeinen Formel ist: 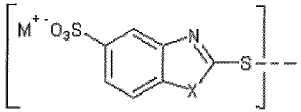
wobei:
X aus der Gruppe bestehend aus -NH-, -O-, -S- und -NR-ausgewählt wird, wobei R eine C1-C₆ lineare oder verzweigte Alkylgruppe C₁-C₆Alk, oder eine Carbonyl-C₁-C₆ lineare oder verzweigte Alkylgruppe -C(O)-C₁-C₆Alk, vorzugsweise Acylgruppe -C(O)-CH₃,und
M⁺ Wasserstoffion oder ein Alkali, Metallion, vorzugsweise Natriumion Na⁺ ist Und wobei in Formel II:
Y ein Gegenanion darstellt, vorzugsweise ausgewählt aus: Acetat, Nitrat, Lactat, Trifluormethan, Chlorat, Bromid und Sulfonat;
[L₂H⁺]⁺ die protonierte Form des Ligandenanteiles ist, ausgewählt aus: 2-Mercapto-4-(C₁-C₆-Alk)-Pyrimidine, vorzugsweise 2-Mercapto-4-Methylpyrimidine, 2-Mercapto-4-(C₁-C₆Alk) Pyrimidine Hydrochlorid, 8-Mercapto-Quinoline Hydrochlorid, vorzugsweise 2-Mercapto-4-Methylpyrimidine Hydrochloride.
Wobei in beiden Formeln (I) und (II) das Ion Ag⁺ auf das thiolische Schwefelatom von [L₁⁻M⁺] und [L₂H⁺]⁺ jeweils abgestimmt ist.

2. Komplex der Formel (I) nach Anspruch 1, wobei L1 ausgewählt ist aus der Gruppe bestehend aus:
2-Mercapto-5-benzoimidazolsulfonsäure, 2-Mercapto-5-benzoimidazolsulfonsäure-Natriumsalz und 2-Mercapto-5-benzooxazolsulfonsäure.

3. Komplex der Formel (I) oder (II) nach einem der vorhergehenden Ansprüche definiert, in Form einer wässrigen Lösung.

4. Komplex der Formel (I) oder (II) in Form einer wässrigen Lösung nach Anspruch 3, erhalten durch das Auflösen des Komplexes (I) oder (II) der Ansprüche 1-3 in einem wässrigen Medium, ausgewählt aus: sterilem Wasser, physiologischer Kochsalzlösung, ultrareinem Wasser, deionisiertem Wasser, destilliertem Wasser und Wasser zur Injektion (WFI).

5. Komplex der Formel (I) oder (II) in Form einer wässrigen Lösung nach Anspruch 3 oder 4, umfassend 0,003 bis 0,0001, vorzugsweise 0,03 bis 0,001 eines Komplexes der Formel (I) oder (II).

6. Wässrige Formulierung, umfassend den Komplex der Formel (I) oder (II) in Form einer wässrigen Lösung wie in den Ansprüchen 3-5 definiert wird und mindestens ein zusätzliches antibakterielles, antivirales oder antimykotisches Mittel, vorzugsweise ausgewählt aus der Gruppe bestehend aus: Chlorexidindigluconat oder -acetat, Didecyldimethylammoniumkationen, vorzugsweise Chlorid und/oder Mischungen davon.

7. Wässrige Formulierung nach einem der Ansprüche 5 oder 6, umfassend den Komplex der Formel (I) in einer Mischung mit Chlorexidindigluconat oder -acetat und Didecyldimethylammoniumchlorid.

8. Wässrige Formulierung nach einem der Ansprüche 5 oder 6, umfassend den Komplex der Formel (II) in einer Mischung mit Chlorexidindigluconat oder -acetat.

9. Wässrige Formulierung nach Anspruch 8, umfassend: 0,003 bis 0,0001, vorzugsweise 0,03 bis 0,001 Gew/Vol% eines Komplexes (I) oder (II), Chlorexidin im Verhältnis 0,1 bis 1 Gew/Vol%, vorzugsweise 0,2 bis 0,6% und Didecylammoniumkation 0,2 bis 2 Gew/Vol%, vorzugsweise 0,5 bis 1,5 Gew/Vol%.

10. Pharmazeutische Zusammensetzung, umfassend den Komplex der Formel (I) oder (II), wie nach einem der Ansprüche 1 bis 5 definiert wird, in einer Mischung mit mindestens einem physiologisch verträglichen Träger und/oder Hilfsstoff und gegebenenfalls auch in einer Mischung mit mindestens einem weiteren antimikrobischen, antiviralen, antimykotischen Mittel.

11. Pharmazeutische Zusammensetzung nach Anspruch 10 zur topischen Verabreichung.

12. Pharmazeutische Zusammensetzung nach Anspruch 11 in Form von Creme, Gel oder Schaum.

13. Verwendung des Komplexes (I) oder (II), wie in einem der Ansprüche 1 bis 5 definiert, als Desinfektionsmittel für Oberflächen, häusliche oder medizinische Gegenstände.

14. Komplex der Formel (I) oder (II) wie in einem der Ansprüche 1 bis 5 definiert wird, zur Verwendung als Arzneimittel.

15. Komplex der Formel (I) oder (II) zur Verwendung, nach Anspruch 13 oder 14 als antimikrobisches, antivirales oder antimykotisches Mittel.

16. Komplex der Formel (I) oder (II) zur Verwendung, nach den Ansprüchen 14-15 als antibakterielles Mittel gegen die Bakterien, ausgewählt aus: Legionellapneumophila, Pseudomonas aeruginosa, Staphilococcus aureus, Enterococcus faecalis, Escherichia coli, Salmonella enteridis D1, Listeria monocytogenes, Neisseria gonorrhoeae, Neisseria meningitidis,Vibrio cholerae, MRSA, Clostridium Diff, Acinetobatcter Baumanii, Mycobacteriuni terrae, Mycobacteriuni avium und Bacillus subtilis.

17. Komplex der Formel (I) oder (II) zur Verwendung, nach den Ansprüchen 14-15 als antimykotikes Mittel gegen: Candida albicans und Aspergillus niger fungi oder als antivirales Mittel gegen die Viren ausgewählt aus: A-H1N1, Adenovirus, Poliovirus, Citomegalovirus, Enterovirus, Herpes Virus, Hepatitis-A-Virus, Hepatitis-B-Virus, Humanen Immundefizienz-Virus (HIV), Varicella Zoster Virus, Aviaria Virengruppe und SARS Corona-Virus.

## Revendications

1. Complexe d'argent de formule générale (I) ou (II) :
Ag⁺---[L₁⁻ M⁺] (I)
Ag⁺---[L₂H⁺]⁺Y⁻] (II)
où :
dans la formule I :
[L₁⁻ M⁺] est un fragment de formule générale : 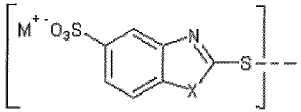 où :
X est choisi dans le groupe constitué par : -NH-, -O-, -S- et -NR-, dans lequel R est un groupe alkyle C₁-C₆Alk linéaire ou ramifié en C1-C₆ ou un groupe alkyle -C(O)-C₁-C₆AIk linéaire ou ramifié en -C₁-C₆ de carbonyle, de préférence un groupe acyle - C(O)-CH₃, et
M⁺ est un ion hydrogène ou un ion métal alcalin, de préférence un ion sodium Na⁺ et où dans la formule II :
Y- représente un contre-anion, de préférence choisi à partir de : acétate, nitrate, lactate, trifluorométhane, chlorate, bromure et sulfonate ;
[L₂H⁺]⁺ est la forme protonée du fragment de ligand choisi à partir de : 2-mercapto-4-(C₁-C₆-Alk)-pyrimidine, de préférence 2-mercapto-4-méthylpyrimidine, 2-mercapto-4-(C₁-C₆Alk) chlorhydrate de pyrimidine, 8-mercapto-chlorhydrate de quinoléine, de préférence 2-mercapto-4-chlorhydrate de méthylpyrimidine.
où dans les deux formules (I) et (II), l'ion Ag⁺ est coordonné à l'atome de soufre thiolique respectivement de [L₁⁻ M⁺] et de [L₂H⁺]⁺.

2. Complexe de formule (I) selon la revendication 1, dans lequel L1 est choisi dans le groupe comprenant :
l'acide sulfonique de 2-Mercapto-5-benzoïmidazole, le sodique de l'acide sulfonique de 2-mercapto-5-benzoïmidazole et l'acide sulfonique de 2-mercapto-5-benzoxazole.

3. Complexe de formule (I) ou (II) tel que défini dans l'une quelconque des revendications précédentes, sous forme d'une solution aqueuse.

4. Complexe de formule (I) ou (II) sous forme d'une solution aqueuse selon la revendication 3, obtenue par dissolution du complexe (I) ou (II) selon les revendications 1-3 dans un milieu aqueux choisi à partir de : eau stérile, solution physiologique salée, eau ultra pure, eau désionisée, eau distillée et eau pour injection (EPI).

5. Complexe de formule (I) ou (II) sous forme d'une solution aqueuse selon la revendication 3 ou 4, comprenant de 0,003 à 0,0001, de préférence de 0,03 à 0,001 d'un complexe de formule (I) ou (II).

6. Formulation aqueuse comprenant le complexe de formule (I) ou (II) sous forme d'une solution aqueuse tel que défini dans les revendications 3-5, et au moins un agent antibactérien, antiviral ou antimycosique supplémentaire, de préférence choisi dans le groupe composé de : chlorhexidine digluconate ou acétate, cations de didécyldiméthylammonium, de préférence de chlorure et/ou leurs mélanges.

7. Formulation aqueuse selon l'une quelconque des revendications 5 ou 6, comprenant le complexe de formule (I) dans un additif avec du chlorhexidine digluconate ou de l'acétate et du chlorure de didécyldiméthylammonium.

8. Formulation aqueuse selon l'une quelconque des revendications 5 ou 6, comprenant le complexe de formule (II) dans un additif avec du chlorhexidine digluconate ou de l'acétate.

9. Formulation aqueuse selon la revendication 8 comprenant: de 0,003 à 0,0001, de préférence de 0,03 à 0,001 en % p/v d'un complexe (I) ou (II), du chlorhexidine dans un rapport compris entre 0,1 et 1 en % p/v, de préférence de 0,2 à 0,6%, et un cation de didécylammonium de 0,2 à 2 en % p/v, de préférence de 0,5 à 1,5 en % p/v.

10. Composition pharmaceutique comprenant le complexe de formule (I) ou (II) tel que défini selon l'une quelconque des revendications 1-5, dans un additif comportant au moins un vecteur et/ou un excipient physiologiquement acceptable(s) et éventuellement aussi dans un additif comportant au moins un agent antibactérien, antiviral ou antimycosique supplémentaire.

11. Composition pharmaceutique selon la revendication 10, destinée à une administration topique.

12. Composition pharmaceutique selon la revendication 11, sous forme de crème, de gel ou de mousse.

13. Utilisation du complexe (I) ou (II) tel que défini dans l'une quelconque des revendications 1-5 comme agent désinfectant pour surfaces, objets domestiques ou médicaux.

14. Complexe de formule (I) ou (II) tel que défini dans l'une quelconque des revendications 1-5, pour une utilisation comme médicament.

15. Complexe de formule (I) ou (II) pour une utilisation, selon les revendications 13 ou 14, comme agent antibactérien, antiviral ou antimycosique.

16. Complexe de formule (I) ou (II) pour une utilisation, selon les revendications 14-15, comme agent antibactérien contre les bactéries choisies parmi : Legionella pneumophila, Pseudomonas aeruginosa, staphylocoque doré, Enterococcus faecalis, Escherichia coli, Salmonella enteritis D1, Listeria monocytogenes, Neisseria gonorrhoeae, Neisseria meningitidis, Vibrio cholerae, SDRM, Clostridium difficile, Acinetobacter Baumanii, Mycobacterium terrae, tuberculose aviaire et bacillus subtilis.

17. Complexe de formule (I) ou (II) pour une utilisation, selon les revendications 14-15, comme agent antimycosique contre : candida albicans et les champignons aspergillus niger ou comme agent antiviral contre les virus choisis parmi : A-H1N1, Adénovirus, Poliovirus, Cytomégalovirus, Entérovirus, herpèsvirus, virus de l'Hépatite A, virus de l'Hépatite B, virus de l'immunodéficience humaine (VIH), virus de la varicelle et du zona, groupe des virus aviaires et coronavirus SRAS.
